# EUROPÄISCHE PATENTSCHRIFT

(11) **EP 2 866 773 B1**
(45) Veröffentlichungstag und Bekanntmachung des Hinweises auf die Patenterteilung: **09.08.2017**
(21) Anmeldenummer: 13723478.7
(22) Anmeldetag: 15.05.2013
(51) Int. Cl.: A61Q 5/04, A61K 8/898, A61K 8/19, A45D 7/04, A45D 7/06, A45D 7/00

(54) **VERFAHREN ZUR CHEMISCHEN GLÄTTUNG VON HUMANHAAREN II**
METHOD FOR CHEMICAL SMOOTHING OF HUMAN HAIRS II
PROCEDE DE LISSAGE CHIMIQUE DE CHEVEUX HUMAINS II

(30) Priorität: 29.06.2012 DE 102012211265
(43) Veröffentlichungstag der Anmeldung: 06.05.2015
(73) Patentinhaber: Henkel AG & Co. KGaA, 40589 Düsseldorf (DE)
(72) Erfinder: SCHULZE ZUR WIESCHE, Erik, 20144 Hamburg (DE); SCHWARTZ, Stephan, 22880 Wedel (DE); RAUTENBERG-GROTH, Birgit, 25479 Ellerau (DE)
(86) Internationale Anmeldenummer: PCT/EP2013/059981
(87) Internationale Veröffentlichungsnummer: WO 2014/000951

(56) Entgegenhaltungen:
- JP-A- H02 250 814
- US-A- 4 324 263
- US-A1- 2003 115 685
- US-A1- 2012 114 584

## Beschreibung

Die Erfindung betrifft das technische Gebiet der Umformung keratinhaltiger Fasern, insbesondere menschlicher Haare. Gegenstand der Erfindung ist ein verbessertes Verfahren zur Umformung keratinhaltiger Fasern, insbesondere menschlicher Haare. Als keratinhaltige Fasern können prinzipiell alle tierischen Haare, z.B. Wolle, Roßhaar, Angorahaar, Pelze, Federn und daraus gefertigte Produkte oder Textilien eingesetzt werden. Bevorzugt wird die Erfindung jedoch im Rahmen einer Haarumformung, insbesondere der Glättung krauser menschlicher Haare sowie daraus gefertigter Perücken, eingesetzt.

Eine dauerhafte Verformung keratinhaltiger Fasern wird üblicherweise derart durchgeführt, dass man die Faser mechanisch verformt und die Verformung durch geeignete Hilfsmittel festlegt. Vor und/oder nach dieser Verformung behandelt man die Faser mit einer keratinreduzierenden Zubereitung. Nach einem Spülvorgang wird die Faser dann in dem so genannten Fixierschritt mit einer Oxidationsmittelzubereitung behandelt, gespült und nach oder während des Fixierschritts von den Verformungshilfsmitteln (z.B. Wicklern, Papilloten) befreit. Wenn als keratinreduzierende Komponente ein Merkaptan, z.B. Ammoniumthioglykolat, verwendet wird, spaltet dieses einen Teil der Disulfid-Brücken des Keratin-Moleküls zu -SH-Gruppen, so dass es zu einer Erweichung der Keratinfaser kommt. Bei der späteren oxidativen Fixierung werden erneut Disulfid-Brücken im Haarkeratin geknüpft, so dass das Keratingefüge in der vorgegebenen Verformung fixiert wird. Alternativ ist es bekannt, zur Haarverformung anstelle der Merkaptane Sulfit zu verwenden. Durch Hydrogensulfit-Lösungen und/oder Sulfit-Lösungen und/oder Disulfit-Lösungen werden Disulfid-Brücken des Keratins in einer Sulfitolyse nach der Gleichung

R-S-S-R +HSO₃⁽⁻⁾ → R-SH +R-S-SO₃⁽⁻⁾

gespalten und auf diese Weise eine Erweichung der Keratinfaser erreicht. Hydrogensulfit-, sulfit- bzw. disulfithaltige Reduktionsmittel weisen nicht den starken Eigengeruch der merkaptanhaltigen Mittel auf. Die Spaltung kann wie zuvor geschildert in einem Fixierschritt mit Hilfe eines Oxidationsmittels unter Bildung von neuen Disulfid-Brücken wieder rückgängig gemacht werden.

Die permanente Glättung keratinhaltiger Fasern wird analog durch den Einsatz von keratinreduzierenden und -oxidierenden Zusammensetzungen erzielt. In einem entsprechenden Verfahren wird das krause Haar entweder auf Wickler mit einem großen Durchmesser von üblicherweise mehr als 15 mm gewickelt oder das Haar unter Einwirkung der keratinreduzierenden Zusammensetzung glattgekämmt. Anstelle des Wicklers ist es auch möglich, die Faser auf ein Glättungsboard glattzulegen. Glättungsboarde sind üblicherweise rechteckige Tafeln z.B. aus Kunststoff. Vorzugsweise ist die Faser dabei mit der keratinreduzierenden Zubereitung benetzt.

Im Allgemeinen haben die bekannten Umformungsverfahren, besonders bei der Glättung, den Nachteil, daß sich die keratinhaltige Faser elektrostatisch auflädt. Darüber hinaus führt die Behandlung mit Umformumgsmitteln zu einer gesteigerten Hydrophilität der Haare, was die Frisierbarkeit erschwert und den Griff, die Kämmbarkeit sowie den Glanz verschlechtert.

Aufgabe der Erfindung ist es daher, ein Umformungsverfahren für keratinhaltige Fasern, insbesondere für menschliches Haar, bereitzustellen, das ein sehr gutes und dauerhaftes Umformungsergebnis liefert und gleichzeitig die elektrostatische Aufladung sowie Hydrophilierung des Haares minimiert, die Faser pflegt sowie die Struktur der Faser schont.

Überraschenderweise wurde gefunden, daß eine Vorbehandlung der Fasern mit speziellen Vorbehandlungsmitteln die negativen Folgen der Umformung deutlich minimiert und insbesondere die elektrostatische Aufladung sowie Hydrophilierung des Haares minimiert. Durch den Einsatz speziell auf die Vorbehandlung abgestimmter Umformungsmittel wird auch das Umformungsergebnis deutlich verbessert.

Gegenstand der vorliegenden Erfindung ist in einer ersten Ausführungsform ein Verfahren zur Umformung, insbesondere zur Glättung, keratinhaltiger Fasern, insbesondere menschlicher Haare, in welchem
(i) ein Vorbehandlungsmittel, enthaltend aminofunktionelle(s) Silikon(e) mit terminale(r/n) Hydroxygruppe(n) auf die keratinischen Fasern aufgetragen und dort belassen wird,
(ii) die Fasern optional getrocknet werden,
(iii) die Fasern im feuchten oder getrockneten Zustand mit einem Umformungsmittel behandelt werden, welches - bezogen auf das Gewicht des Umformungsmittels - 0,05 bis 20 Gew.-% Alkalimetallhydroxid(e) enthält,
(iv) die Fasern optional während der Umformungsbehandlung mit einem Kamm oder einer Bürste geglättet werden,
(v) die Fasern mit einem Shampoo, welches einen pH von 2,5 bis 6,5 aufweist, shampooniert, gespült und neutralisiert werden,
(vi) die Fasern gegebenenfalls nachfolgend zusätzlich unter der Einwirkung von Wärme mechanisch verformt werden.

Im erfindungsgemäßen Verfahren wird zunächst ein Vorbehandlungsmittel, enthaltend aminofunktionelle(s) Silikon(e) mit terminale(r/n) Hydroxygruppe(n) auf die keratinischen Fasern aufgetragen und dort belassen.
Das in Schritt (i) aufgetragene Vorbehandlungsmittel wird nicht ausgespült, sondern verbleibt auf der Faser. Die Faser kann in Schritt (ii) optional getrocknet werden, was bei längeren Einwirkzeiten des Vorbehandlungsmittels durch Lufttrocknung erfolgen kann. Bei kürzeren Applikationszeiten kann das Haar beispielsweise mit einem Handtuch frottiert werden. Nach Abschluß des Frottierschritts bleibt eine fühlbare Restfeuchtigkeit im Haar zurück.

Die im erfindungsgemäßen Verfahren eingesetzten Vorbehandlungsmittel enthalten aminofunktionelle(s) Silikon(e) mit terminale(r/n) Hydroxygruppee(n). Solche Silicone können z.B. durch die Formel

M(RₐO_{b}SiO_{(4-a-b)/2)x}(R_{c}SiO_{(4-c)/2})_{y}M

beschrieben werden, wobei in der obigen Formel R ein Kohlenwasserstoff oder ein Kohlenwasserstoffrest mit 1 bis etwa 6 Kohlenstoffatomen ist, Q ein polarer Rest der allgemeinen Formel -R¹HZ ist, worin R¹ eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und Z ein organischer, aminofunktioneller Rest ist, der mindestens eine aminofunktionelle Gruppe enthält; "a" Werte im Bereich von etwa 0 bis etwa 2 annimmt, "b" Werte im Bereich von etwa 1 bis etwa 3 annimmt, "a" + "b" kleiner als oder gleich 3 ist, und "c" eine Zahl im Bereich von etwa 1 bis etwa 3 ist, und x eine Zahl im Bereich von 1 bis etwa 2.000, vorzugsweise von etwa 3 bis etwa 50 und am bevorzugtesten von etwa 3 bis etwa 25 ist, und y eine Zahl im Bereich von etwa 20 bis etwa 10.000, vorzugsweise von etwa 125 bis etwa 10.000 und am bevorzugtesten von etwa 150 bis etwa 1.000 ist, und M eine geeignete Silicon-Endgruppe mit Hydroxyfunktion ist, vorzugsweise Hydroxy-Dimethylsiloxy. Nicht einschränkende Beispiele der durch R repräsentierten Reste schließen Alkylreste, wie Methyl, Ethyl, Propyl, Isopropyl, Isopropyl, Butyl, Isobutyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R ein Alkylrest, der 1 bis etwa 6 Kohlenstoffatomen enthält, und am bevorzugtesten ist R Methyl. Beispiele von R¹ schließen Methylen, Ethylen, Propylen, Hexamethylen, Decamethylen,-CH₂CH(CH₃)CH₂-, Phenylen, Naphthylen, -CH₂CH₂SCH₂CH ₂-, -CH₂CH₂OCH₂-, -OCH₂CH₂-,-OCH₂ CH₂CH₂-, -CH₂CH(CH₃)C(O)OCH₂-, -(CH₂)₃ CC(O)OCH₂CH₂-, -C₆H₄C₆H₄-, -C₆H₄CH₂C₆H₄-; und -(CH₂)₃C(O)SCH₂CH₂- ein.

Z ist ein organischer, aminofunktioneller Rest, enthaltend mindestens eine funktionelle Aminogruppe. Eine mögliche Formel für Z ist NH(CH₂)_{z}NH₂, worin z 1 oder mehr ist. Eine andere mögliche Formel für Z ist -NH(CH₂)_{z}(CH₂)_{zz}NH, worin sowohl z als auch zz unabhängig 1 oder mehr sind, wobei diese Struktur Diamino-Ringstrukturen umfaßt, wie Piperazinyl. Z ist am bevorzugtesten ein -NHCH₂CH₂NH₂-Rest. Eine andere mögliche Formel für Z ist - N(CH₂)_{z}(CH₂)_{zz}NX₂ oder -NX₂, worin jedes X von X₂ unabhängig ausgewählt ist aus der Gruppe bestehend aus Wasserstoff und Alkylgruppen mit 1 bis 12 Kohlenstoffatomen, und zz 0 ist.

Q ist am bevorzugtesten ein polarer, aminfunktioneller Rest der Formel -CH₂CH₂CH₂NHCH₂CH₂NH ₂. In den Formeln nimmt "a" Werte im Bereich von etwa 0 bis etwa 2 an, "b" nimmt Werte im Bereich von etwa 2 bis etwa 3 an, "a" + "b" ist kleiner als oder gleich 3, und "c" ist eine Zahl im Bereich von etwa 1 bis etwa 3. Das molare Verhältnis der RₐQ_{b} SiO_{(4-a-b)/2}-Einheiten zu den R_{c}SiO _{(4-c)/2}-Einheiten liegt im Bereich von etwa 1 : 2 bis 1 : 65, vorzugsweise von etwa 1 : 5 bis etwa 1 : 65 und am bevorzugtesten von etwa 1 : 15 bis etwa 1 : 20. Werden ein oder mehrere Silicone der obigen Formel eingesetzt, dann können die verschiedenen variablen Substituenten in der obigen Formel bei den verschiedenen Siliconkomponenten, die in der Siliconmischung vorhanden sind, verschieden sein.

In erfindungsgemäßen Verfahren können beispielsweise aminofunktionelle Silikone der Formel (Si-II) eingesetzt werden:

R'ₐG₃₋ₐ-Si(OSiG₂)ₙ-(OSiG_{b}R'_{2-b})ₘ-O-SiG₃₋ₐ-OH (Si-II),

worin bedeutet:
- G ist-H, eine Phenylgruppe, -OH, -O-CH₃, -CH₃, -O-CH₂CH₃, -CH₂CH₃, -O-CH₂CH₂CH₃,-CH₂CH₂CH₃, -O-CH(CH₃)₂, -CH(CH₃)₂, -O-CH₂CH₂CH₂CH₃,-CH₂CH₂CH₂CH₃, -O-CH₂CH(CH₃)₂, -CH₂CH(CH₃)₂, -O-CH(CH₃)CH₂CH₃,-CH(CH₃)CH₂CH₃, -O-C(CH₃)₃, -C(CH₃)₃ ;
- a steht für eine Zahl zwischen 0 und 3, insbesondere 0;
- b steht für eine Zahl zwischen 0 und 1, insbesondere 1,
- m und n sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R' ist ein monovalenter Rest ausgewählt aus
   ∘ -Q-N(R")-CH₂-CH₂-N(R")₂
   ∘ -Q-N(R")₂
   ∘ -Q-N⁺(R")₃A⁻
   ∘ -Q-N⁺H(R")₂ A⁻
   ∘ -Q-N⁺H₂(R")A⁻
   ∘ -Q-N(R")-CH₂-CH₂-N⁺R"H₂A⁻,
wobei jedes Q für eine chemische Bindung, -CH₂-, -CH₂-CH₂-, -CH₂CH₂CH₂-, -C(CH₃)₂-, -CH₂CH₂CH₂CH₂-, -CH₂C(CH₃)₂-, -CH(CH₃)CH₂CH₂- steht,
R" für gleiche oder verschiedene Reste aus der Gruppe -H, -Phenyl, -Benzyl, -CH₂-CH(CH₃)Ph, der C₁₋₂₀-Alkylreste, vorzugsweise -CH₃, -CH₂CH₃, -CH₂CH₂CH₃,-CH(CH₃)₂, -CH₂CH₂CH₂H₃, -CH₂CH(CH₃)₂, -CH(CH₃)CH₂CH₃, -C(CH₃)₃, steht und A ein Anion repräsentiert, welches vorzugsweise ausgewählt ist aus Chlorid, Bromid, lodid oder Methosulfat.

In erfindungsgemäßen Verfahren dieser Ausführungsform können vorzugsweise aminofunktionelle Silikone der Formel (Si-IIa) eingesetzt werden: 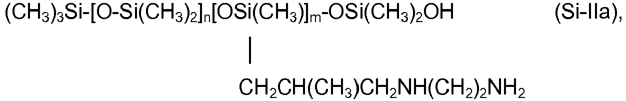 worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Besonders bevorzugt sind auch erfindungsgemäße Mittel, die ein aminofunktionelles Silikon der Formel (Si-IIb) 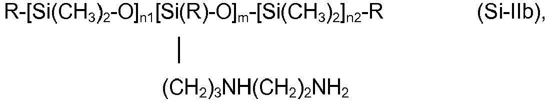 enthalten, worin R für -OH, -O-CH₃ oder eine -CH₃-Gruppe steht und m, n1 und n2 Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt. Erfindungsgemäß steht dabei mindestens ein R für -OH.

Diese Silicone werden nach der INCI-Deklaration als Amodimethicone bezeichnet.

Unabhängig davon, welche aminofunktionellen Silicone eingesetzt werden, sind erfindungsgemäße Mittel bevorzugt, die ein aminofunktionelles Silikon enthalten dessen Aminzahl oberhalb von 0,25 meq/g, vorzugsweise oberhalb von 0,3 meq/g und insbesondere oberhalb von 0,4 meq/g liegt. Die Aminzahl steht dabei für die Milli-Äquivalente Amin pro Gramm des aminofunktionellen Silicons. Sie kann durch Titration ermittelt und auch in der Einheit mg KOH/g angegeben werden.

Die Einsatzmenge des/der aminofunktionellen Silikon(s/e) mit terminale(r/n) Hydroxygruppe(n) in dem im erfindungsgemäßen Verfahren eingesetzten Vorbehandlungsmittel kann variieren, bevorzugte Verfahren sind dadurch gekennzeichnet, daß das Vorbehandlungsmittel bezogen auf sein Gewicht 0,00001 bis 10 Gew.-%, vorzugsweise 0,0001 bis 7,5 Gew.-%, weiter bevorzugt 0,001 bis 5 Gew.-%, noch weiter bevorzugt 0,01 bis 2,5 Gew.-% und insbesondere 0,1 bis 1 Gew.-% aminofunktionelle(s) Silikon(e) mit terminale(r/n) Hydroxygruppee(n) enthält.

Einige spezielle aminofunktionelle(s) Silikon(e) mit terminale(r/n) Hydroxygruppe(n) haben sich im erfindungsgemäßen Verfahren als besonders geeignet herausgestellt. Diese werden nachstehend beschrieben.

Erfindungsgemäß bevorzugt Verfahren sind dadurch gekennzeichnet, daß im Schritt (i) ein Vorbehandlungsmittel aufgetragen wird, welches bezogen auf sein Gewicht 0,01 bis 5 Gew.-%, vorzugsweise 0,025 bis 2,5 Gew.-% weiter bevorzugt 0,05 bis 1,5 Gew.-%, noch weiter bevorzugt 0,075 bis 1 Gew.-% und insbesondere 0,1 bis 0,25 Gew.-% mindestens eines Silikons der Formel (I) 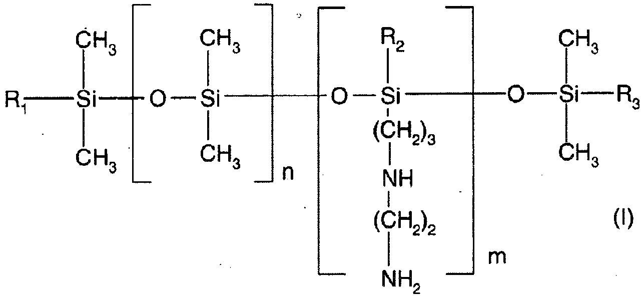 enthält, in der
- m und n bedeuten Zahlen, die so gewählt sind, daß die Summe (n + m) im Bereich von 1 bis 1000 liegt,
- n ist eine Zahl im Bereich von 0 bis 999 und m ist eine Zahl im Bereich von 1 bis 1000,
- R1, R2 und R3, die gleich oder verschieden sind, bedeuten eine Hydroxygruppe oder eine C1-4-Alkoxygruppe,
- wobei mindestens eine der Gruppen R1 bis R3 eine Hydroxygruppe bedeutet;

Weitere erfindungsgemäß bevorzugte Verfahren sind dadurch gekennzeichnet, daß im Schritt (i) ein Vorbehandlungsmittel aufgetragen wird, welches bezogen auf sein Gewicht 0,01 bis 5 Gew.-%, vorzugsweise 0,025 bis 2,5 Gew.-% weiter bevorzugt 0,05 bis 1,5 Gew.-%, noch weiter bevorzugt 0,075 bis 1 Gew.-% und insbesondere 0,1 bis 0,25 Gew.-% mindestens eines Silikons der Formel (II) 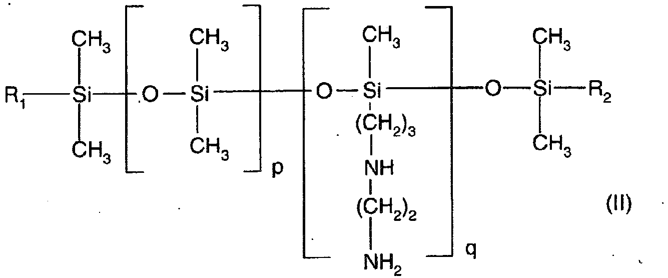 enthält, in der
- p und q bedeuten Zahlen, die so gewählt sind, daß die Summe (p + q) im Bereich von 1 bis 1000 liegt,
- p ist eine Zahl im Bereich von 0 bis 999 und q ist eine Zahl im Bereich von 1 bis 1000,
- R1 und R2, die verschieden sind, bedeuten eine Hydroxygruppe oder eine C1-4-Alkoxygruppe, wobei mindestens eine der Gruppen R1 bis R2 eine Hydroxygruppe bedeutet.

Die Silikone der Formeln (I) und (II) unterscheiden sich durch die Gruppierung am Si-Atom, das die stickstoffhaltige Gruppe trägt: In Formel (I) bedeutet R2 eine Hydroxygruppe oder eine C1-4-Alkoxygruppe, während der Rest in Formel (II) eine Methylgruppe ist. Die einzelnen Si-Gruppierungen, die mit den Indices m und n bzw. p und q gekennzeichnet sind, müssen nicht als Blöcke vorliegen, vielmehr können die einzelnen Einheiten auch statistisch verteilt vorliegen, d.h. in den Formeln (I) und (II) ist nicht zwingend jedes R1-Si(CH₃)₂-Gruppe an eine -[O-Si(CH₃)₂]-Gruppierung gebunden.

Als besonders wirkungsvoll im Hinblick auf die gewünschten Effekte haben sich im erfindungsgemäßen Verfahren Vorbehandlungsmittel erwiesen, die mindestens ein Silikon der Formel (III) enthalten: 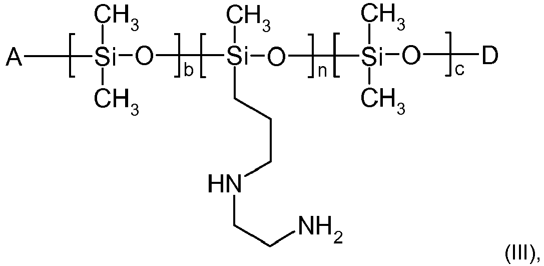 in der
- A: für eine Gruppe -OH, -O-Si(CH₃)₃,-O-Si(CH₃)₂OH -O-Si(CH₃)₂OCH₃ steht,
- D: für eine Gruppe -H, -Si(CH₃)₃,-Si(CH₃)₂OH, -Si(CH₃)₂OCH₃ steht,
b, n und c für ganze Zahlen zwischen 0 und 1000 stehen, mit den Maßgaben
- n > 0 und b + c > 0
- mindestens eine der Bedingungen A = -OH bzw. D = -H ist erfüllt.

Erfindungsgemäße Verfahren, bei denen im Schritt (i) ein Vorbehandlungsmittel aufgetragen wird, welches bezogen auf sein Gewicht 0,01 bis 5 Gew.-%, vorzugsweise 0,025 bis 2,5 Gew.-% weiter bevorzugt 0,05 bis 1,5 Gew.-%, noch weiter bevorzugt 0,075 bis 1 Gew.-% und insbesondere 0,1 bis 0,25 Gew.-% mindestens eines Silikons der Formel (III) enthält: 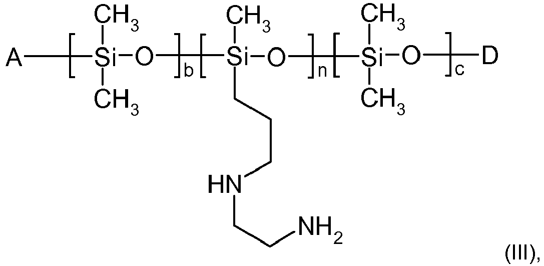 in der
- A: für eine Gruppe -OH, -O-Si(CH₃)₃,-O-Si(CH₃)₂OH ,-O-Si(CH₃)₂OCH₃ steht,
- D: für eine Gruppe -H, -Si(CH₃)₃,-Si(CH₃)₂OH, -Si(CH₃)₂OCH₃ steht,
- b, n und c: für ganze Zahlen zwischen 0 und 1000 stehen,
mit den Maßgaben
- n > 0 und b + c > 0
- mindestens eine der Bedingungen A = -OH bzw. D = -H ist erfüllt,
sind demnach erfindungsgemäß bevorzugt.

In der vorstehend genannten Formel (III) sind die einzelnen Siloxaneinheiten mit den Indices b, c und n statistisch verteilt, d.h. es muß sich nicht zwingend um Blockcopolymere handeln.

Weitere besonders geeignete Silikone sind 4-morpholinomethyl-substituiert. Erfindungsgemäße Verfahren, bei denen das Vorbehandlungsmittel bezogen auf sein Gewicht 0,01 bis 5 Gew.-%, vorzugsweise 0,025 bis 2,5 Gew.-% weiter bevorzugt 0,05 bis 1,5 Gew.-%, noch weiter bevorzugt 0,075 bis 1 Gew.-% und insbesondere 0,1 bis 0,25 Gew.-% mindestens eines 4-morpholinomethyl-substituierten Silikons der Formel (IV) enthält, 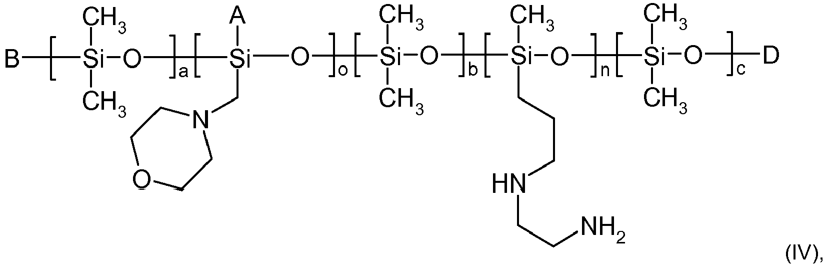 in der
- A: für eine über ein -O- gebundene Struktureinheit (I) 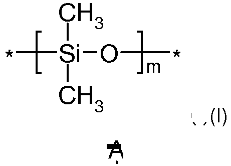 oder einen über ein -O- gebundenen oligomeren oder polymeren Rest enthaltend Struktureinheiten der Formeln (I), oder für -OH steht,
- *: für eine Endgruppe B (Si-gebunden) oder D (O-gebunden) steht,
- B: für eine Gruppe -OH, -O-Si(CH₃)₃,-O-Si(CH₃)₂OH ,-O-Si(CH₃)₂OCH₃ steht,
- D: für eine Gruppe -H, -Si(CH₃)₃,-Si(CH₃)₂OH, -Si(CH₃)₂OCH₃ steht,
a, b und c für ganze Zahlen zwischen 0 und 1000 stehen, mit der Maßgabe a + b + c > 0 m, n und o für ganze Zahlen zwischen 1 und 1000 stehen. mit der Maßgabe, daß mindestens eine der Bedingungen B = -OH bzw. D = -H erfüllt ist, sind besonders bevorzugt.

Strukturformel (IV) soll verdeutlichen, daß die Siloxangruppen n und o nicht zwingend direkt an eine Endgruppierung B bzw. D gebunden sein müssen. Vielmehr gilt in bevorzugten Formeln (IV) a > 0 oder b > 0 und in besonders bevorzugten Formeln (IV) a > 0 und b > 0, d.h. die terminale Gruppierung B bzw. D ist vorzugsweise an eine Dimethylsiloxy-Gruppierung gebunden. Auch in Formel (IV) sind die Siloxaneinheiten a, b, c, n und o vorzugsweise statistisch verteilt. Die durch Formel (IV) dargestellten erfindungsgemäß eingesetzten Silikone können trimethylsilylterminiert sein (D oder B = -Si(CH₃)₃), sie können aber auch zweiseitig dimethylsilylhydroxy- oder einseitig dimethylsilylhydroxy- und dimethylsilylmethoxy-terminiert sein. Im Rahmen der vorliegenden Erfindung besonders bevorzugt eingesetzte Silikone sind ausgewählt aus Silikonen, in denen

| | | |
|---|---|---|
| B = -O-Si(CH₃)₂OH | und | D = -Si(CH₃)₃ |
| B = -O-Si(CH₃)₂OH | und | D = -Si(CH₃)₂OH |
| B = -O-Si(CH₃)₂OH | und | D = -Si(CH₃)₂OCH₃ |
| B = -O-Si(CH₃)₃ | und | D = -Si(CH₃)₂OH |
| B = -O-Si(CH₃)₂OCH₃ | und | D = -Si(CH₃)₂OH |

bedeutet. Diese Silikone führen zu exorbitanten Verbesserungen der Haareigenschaften der mit den erfindungsgemäßen Mitteln behandelten Haare, insbesondere zu einem gravierend verbesserten Schutz bei oxidativer Behandlung. Auch in Formel (IV) kann der Rest A
- für eine über ein -O- gebundene Struktureinheit (i), (ii) oder (iii) oder
- einen über ein -O- gebundenen oligomeren oder polymeren Rest enthaltend Struktureinheiten der Formeln (i), (ii) oder (iii)
- oder die Hälfte eines verbundenen O-Atoms zu einer Struktureinheit (iii) oder für -OH stehen.

Damit wird Formel (IV) präzisiert zu einer der Formeln (IVa), oder (IVf): 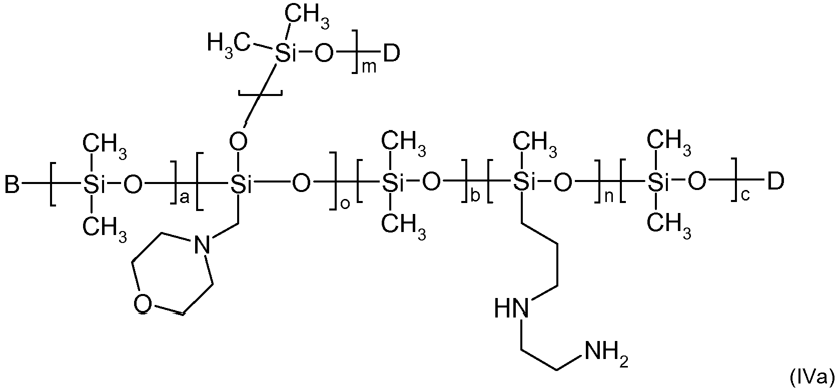 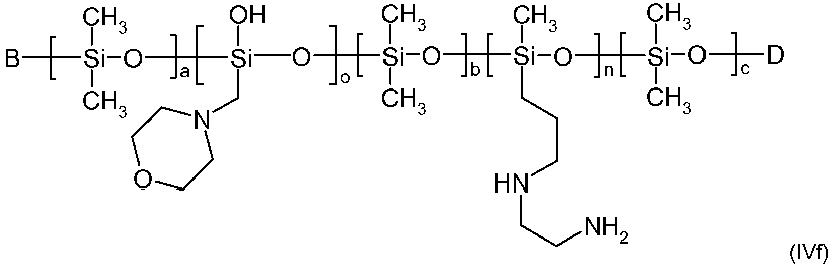 Erfindungsgemäße Vorbehandlungsmittel, die Silikone mit entsprechenden 4-morpholinomethyl-substituierten Silsesquioxan- Teilstrukturen beinhalten, sind erfindungsgemäß bevorzugt, da diese Silikone zu enorm verbessertem Haarschutz vor oxidativer Behandlung führen.

Es ist in erfindungsgemäßen Verfahren der vorstehend genannten Ausführungsform besonders bevorzugt, wenn das Vorbehandlungsmittel es - bezogen auf sein Gewicht - 0,00001 bis 10 Gew.-%, vorzugsweise 0,0001 bis 7,5 Gew.-%, besonders bevorzugt 0,001 bis 5 Gew.-%, weiter bevorzugt 0,01 bis 3 Gew.-% und insbesondere 0,1 bis 1 Gew.-% 4-morpholinomethyl-substituierte(s) Silikon(e) enthält.

Unabhängig von der Art des bzw. der eingesetzten aminofunktionellen Silikon(s/e) mit terminale(r/n) Hydroxygruppee(n) enthalten die im erfindungsgemäßen Verfahren eingesetzten Vorbehandlungsmittel das/die Silikon(e) vorzugsweise in Form einer Emulsion, besonders bevorzugt in Form einer Mikroemulsion. Als besonders bevorzugt haben sich Mikroemulsionen erwiesen, die Fettalkohole als Emulgatoren bzw. Stabilisatoren enthalten, so daß bevorzugte erfindungsgemäße Verfahren dadurch gekennzeichnet sind, daß das Vorbehandlungsmittel in Form einer Mikroemulsion vorliegt, welche Fettalkohol(e) enthält.

Es hat sich gezeigt, daß die Wirkung der im Rahmen des erfindungsgemäßen Verfahrens in den Vorbehandlungsmitteln eingesetzten Silikone noch gesteigert werden kann, wenn bestimmte nichtionische Komponenten ebenfalls in den Vorbehandlungsmitteln eingesetzt werden. Zudem haben diese nichtionischen Komponenten positive Effekte auf die Lagerstabilität der Vorbehandlungsmittel. Nichtionische Komponenten, die hier besonders geeignet sind, sind Ethoxylate von Decanol, Undecanol, Dodecanol, Tridecanol usw.. Als besonders geeignet haben sich ethoxylierte Tridecanole erwiesen, die mit besonderem Vorzug in die im erfindungsgemäßen Verfahren eingesetzten Vorbehandlungsmittel inkorporiert werden. Erfindungsgemäß besonders bevorzugte Verfahren sind dadurch gekennzeichnet, daß das Vorbehandlungsmittel- bezogen auf sein Gewicht - 0,00001 bis 5 Gew.-%, vorzugsweise 0,0001 bis 3,5 Gew.-%, besonders bevorzugt 0,001 bis 2 Gew.-%, weiter bevorzugt 0,01 bis 1 Gew.-% und insbesondere 0,1 bis 0,5 Gew.-% verzweigtes, ethoxyliertes Tridecanol (INCI-Bezeichnung: Trideceth-5) oder α-iso-Tridecyl-ω-hydroxypolyglycolether (INCI-Bezeichnung: Trideceth-10) oder deren Mischungen enthält.

Die im erfindungsgemäßen Verfahren eingesetzten Vorbehandlungsmittel können darüber hinaus weitere übliche Inhaltsstoffe kosmetischer Mittel enthalten.

Wie bereits erwähnt, kann die Faser in Schritt (ii) optional getrocknet werden, was bei längeren Einwirkzeiten des Vorbehandlungsmittels durch Lufttrocknung erfolgen kann. Bei kürzeren Applikationszeiten kann das Haar beispielsweise mit einem Handtuch frottiert werden. Nach Abschluß des Frottierschritts bleibt eine fühlbare Restfeuchtigkeit im Haar zurück. Erfindungsgemäß bevrozugte Verfahren sind dadurch gekennzeichnet, daß die Fasern in Schritt (ii) getrocknet werden.

Im nächsten Schritt (iii) des erfindungsgemäßen Verfahrens wird ein Umformungshilfsmittel auf die Fasern aufgetragen. Erfindungsgemäß enthält das Umformungsmittel - bezogen auf sein Gewicht-0,05 bis 20 Gew.-% Alkalimetallhydroxid(e). Aus Verfügbarkeits- und Kostengründen haben sich Natrium- und Kaliumhydroxid besonders bewährt. Insbesondere bevorzugte erfindungsgemäße Verfahren sind dadurch gekennzeichnet, daß die Fasern in Schritt (iii) mit einem Umformungsmittel behandelt werden, welches - bezogen auf das Gewicht des Umformungsmittels - 0,05 bis 20 Gew.-%, vorzugsweise 0,1 bis 15 Gew.-%, weiter bevorzugt 0,25 bis 10 Gew.-%, noch weiter bevorzugt 0,5 bis 5 Gew.-%, besonders bevorzugt 0,75 bis 3 Gew.-% und insbesondere 1 bis 2 Gew.-% Natriumhydroxid enthält.

Während des Einwirkens des Umformungsmittels können die Fasern mechanisch behandelt, insbesondere gekämmt oder gebürstet werden. Erfindungsgemäß bevorzugte Verfahrens beinhalten dabei einen Schritt (iv), bei dem die Fasern während der Umformungsbehandlung mit einem Kamm oder einer Bürste geglättet werden.

Die beiden in Schritt (i) (Vorbehandlungsmittel) und (iii) (Umformungsmittel) auf die keratinischen Fasern aufgebrachten Mittel werden in Schritt (v) des erfindungsgemäßen Verfahrens von den Fasern entfernt, indem die Fasern mit einem Shampoo, welches einen pH von 2,5 bis 6,5 aufweist, shampooniert, gespült und neutralisiert werden. Durch die leicht saure Behandlung wird der im Zuge des Einsatzes des Umformungsmittels gestiegen pH Wert von ca. 8-11 wieder abgesenkt. Entsprechende Neutralisierungsshampoos mit pH-Werten zwischen 2,5 und 6,5, vorzugsweise zwischen 5,5 und 6,5 können sämtliche üblichen Inhaltsstoffe von Haarreinigungsmitteln enthalten.

Erfindungsgemäß bevorzugte Verfahren sind dabei weiterhin dadurch gekennzeichnet, daß das Shampoo im Behandlungsschritt (v) Phenolsulphonthalein als pH-Indikator enthält.

Unabhängig davon, ob die Haare in Schritt (iv) des erfindungsgemäßen Verfahrens mit Hilfe eines Kammes oder einer Bürste geglättet wurden, kann die Umformung der Keratinfasern durch einen weiteren Verfahrensschritt unterstützt werden. Diese sich an das Shampoonieren anschließende mechanische Verformung kann in Schritt (vi) des erfindungsgemäßen Verfahrens erfolgen, wobei ggf. eine weitere Unterstützung der Umformung durch Wärme erzielt werden kann, beispielsweise durch beheizte Lockenwickler oder - besonders bevorzugt - durch Anwendung eine Glätteisens.

Erfindungsgemäße Verfahren, bei denen die Fasern in Schritt (vi) einer Wärmebehandlung bei einer Temperatur von 50°C bis 350°C (vorzugsweise 80°C bis 280°C, besonders bevorzugt 100°C bis 250°C, weiter bevorzugt 140°C bis 220°C) unterworfen werden, sind erfindungsgemäß bevorzugt.

## Patentansprüche

1. Verfahren zur Umformung, insbesondere zur Glättung, keratinhaltiger Fasern, insbesondere menschlicher Haare, in welchem
(i) ein Vorbehandlungsmittel, enthaltend aminofunktionelle(s) Silikon(e) mit terminale(r/n) Hydroxygruppe(n) auf die keratinischen Fasern aufgetragen und dort belassen wird,
(ii) die Fasern optional getrocknet werden,
(iii) die Fasern im feuchten oder getrockneten Zustand mit einem Umformungsmittel behandelt werden, welches - bezogen auf das Gewicht des Umformungsmittels - 0,05 bis 20 Gew.-% Alkalimetallhydroxid(e) enthält,
(iv) die Fasern optional während der Umformungsbehandlung mit einem Kamm oder einer Bürste geglättet werden,
(v) die Fasern mit einem Shampoo, welches einen pH von 2,5 bis 6,5 aufweist, shampooniert, gespült und neutralisiert werden,
(vi) die Fasern gegebenenfalls nachfolgend zusätzlich unter der Einwirkung von Wärme mechanisch verformt werden.

2. Verfahren nach Anspruch 1, **dadurch gekennzeichnet, daß** im Schritt (i) ein Vorbehandlungsmittel aufgetragen wird, welches bezogen auf sein Gewicht 0,01 bis 5 Gew.-%, vorzugsweise 0,025 bis 2,5 Gew.-% weiter bevorzugt 0,05 bis 1,5 Gew.-%, noch weiter bevorzugt 0,075 bis 1 Gew.-% und insbesondere 0,1 bis 0,25 Gew.-% mindestens eines Silikons der Formel (I) 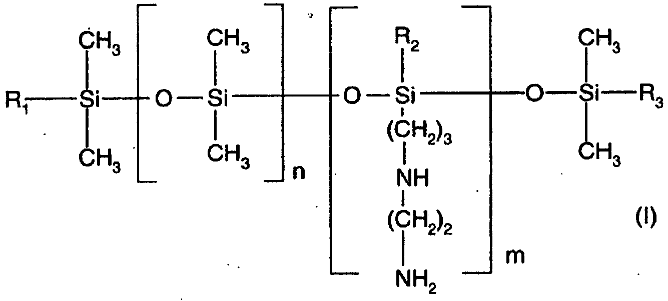 enthält, in der
- m und n bedeuten Zahlen, die so gewählt sind, daß die Summe (n + m) im Bereich von 1 bis 1000 liegt,
- n ist eine Zahl im Bereich von 0 bis 999 und m ist eine Zahl im Bereich von 1 bis 1000,
- R1, R2 und R3, die gleich oder verschieden sind, bedeuten eine Hydroxygruppe oder eine C1-4-Alkoxygruppe,
- wobei mindestens eine der Gruppen R1 bis R3 eine Hydroxygruppe bedeutet;

3. Verfahren nach einem der Ansprüche 1 oder 2, **dadurch gekennzeichnet, daß** im Schritt (i) ein Vorbehandlungsmittel aufgetragen wird, welches bezogen auf sein Gewicht 0,01 bis 5 Gew.-%, vorzugsweise 0,025 bis 2,5 Gew.-% weiter bevorzugt 0,05 bis 1,5 Gew.-%, noch weiter bevorzugt 0,075 bis 1 Gew.-% und insbesondere 0,1 bis 0,25 Gew.-% mindestens eines Silikons der Formel (II) 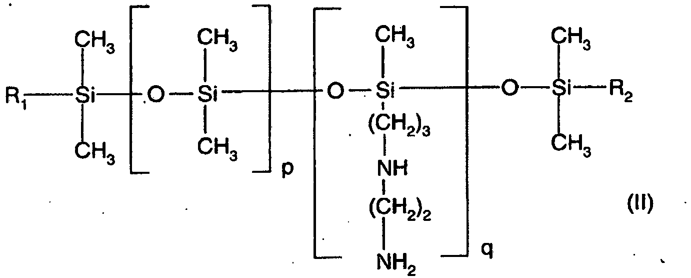 enthält, in der
- p und q bedeuten Zahlen, die so gewählt sind, daß die Summe (p + q) im Bereich von 1 bis 1000 liegt,
- p ist eine Zahl im Bereich von 0 bis 999 und q ist eine Zahl im Bereich von 1 bis 1000,
- R1 und R2, die verschieden sind, bedeuten eine Hydroxygruppe oder eine C1-4-Alkoxygruppe, wobei mindestens eine der Gruppen R1 bis R2 eine Hydroxygruppe bedeutet.

4. Verfahren nach einem der Ansprüche 1 bis 3, **dadurch gekennzeichnet, daß** im Schritt (i) ein Vorbehandlungsmittel aufgetragen wird, welches bezogen auf sein Gewicht 0,01 bis 5 Gew.-%, vorzugsweise 0,025 bis 2,5 Gew.-% weiter bevorzugt 0,05 bis 1,5 Gew.-%, noch weiter bevorzugt 0,075 bis 1 Gew.-% und insbesondere 0,1 bis 0,25 Gew.-% mindestens eines Silikons der Formel (III) enthält: 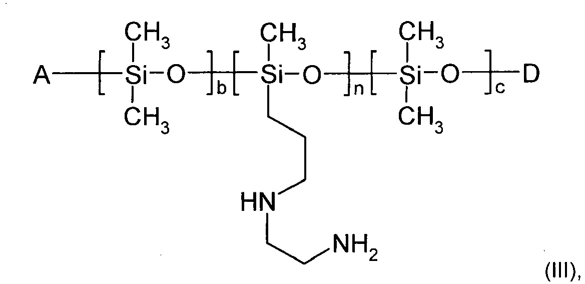 in der
A für eine Gruppe -OH, -O-Si(CH₃)₃,-O-Si(CH₃)₂OH ,-O-Si(CH₃)₂OCH₃ steht,
D für eine Gruppe -H, -Si(CH₃)₃,-Si(CH₃)₂OH, -Si(CH₃)₂OCH₃ steht,
b, n und c für ganze Zahlen zwischen 0 und 1000 stehen, mit den Maßgaben
- n > 0 und b + c > 0
- mindestens eine der Bedingungen A = -OH bzw. D = -H ist erfüllt.

5. Verfahren nach einem der Ansprüche 1 bis 4, **dadurch gekennzeichnet, daß** im Schritt (i) ein Vorbehandlungsmittel aufgetragen wird, welches bezogen auf sein Gewicht 0,01 bis 5 Gew.-%, vorzugsweise 0,025 bis 2,5 Gew.-% weiter bevorzugt 0,05 bis 1,5 Gew.-%, noch weiter bevorzugt 0,075 bis 1 Gew.-% und insbesondere 0,1 bis 0,25 Gew.-% mindestens eines 4-morpholinomethyl-substituierten Silikons der Formel (IV) enthält: 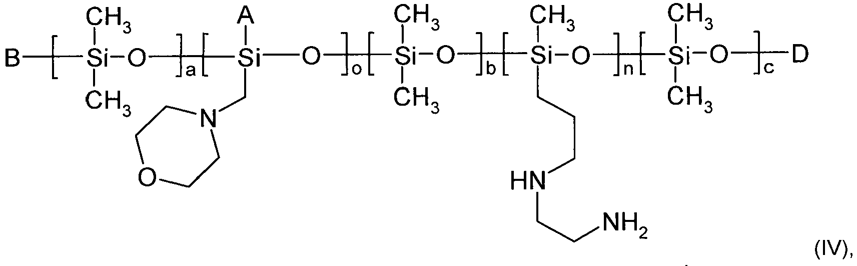 in der
A für eine über ein -O- gebundene Struktureinheit (I) 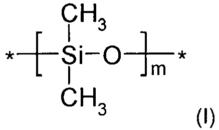 oder einen über ein -O- gebundenen oligomeren oder polymeren Rest enthaltend Struktureinheiten der Formeln (I) oder für -OH steht,
* für eine Endgruppe B (Si-gebunden) oder D (O-gebunden) steht,
B für eine Gruppe -OH, -O-Si(CH₃)₃,-O-Si(CH₃)₂OH -O-Si(CH₃)₂OCH₃ steht,
D für eine Gruppe -H, -Si(CH₃)₃,-Si(CH₃)₂OH, -Si(CH₃)₂OCH₃ steht,
a, b und c für ganze Zahlen zwischen 0 und 1000 stehen, mit der Maßgabe a + b + c > 0
m, n und o für ganze Zahlen zwischen 1 und 1000 stehen,
mit der Maßgabe, daß mindestens eine der Bedingungen B = -OH bzw. D = -H erfüllt ist.

6. Verfahren nach einem der Ansprüche 1 bis 5, **dadurch gekennzeichnet, daß** im Schritt (i) ein Vorbehandlungsmittel aufgetragen wird, welches bezogen auf sein Gewicht 0,01 bis 5 Gew.-%, vorzugsweise 0,025 bis 2,5 Gew.-% weiter bevorzugt 0,05 bis 1,5 Gew.-%, noch weiter bevorzugt 0,075 bis 1 Gew.-% und insbesondere 0,1 bis 0,25 Gew.-% mindestens eines 4-morpholinomethyl-substituierten Silikons der Formeln (IVa), oder (IVf) 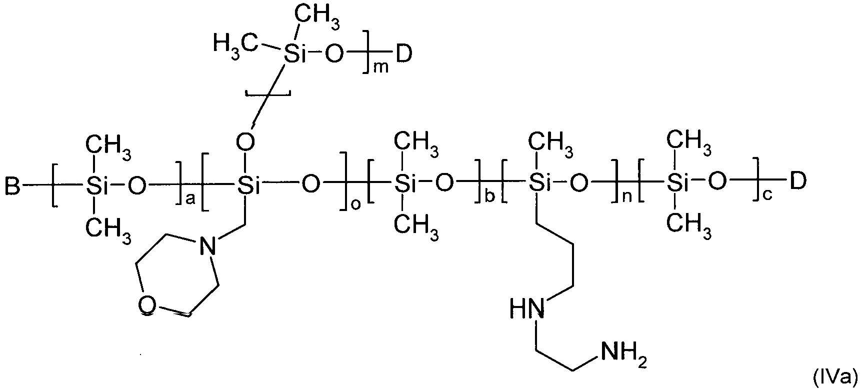 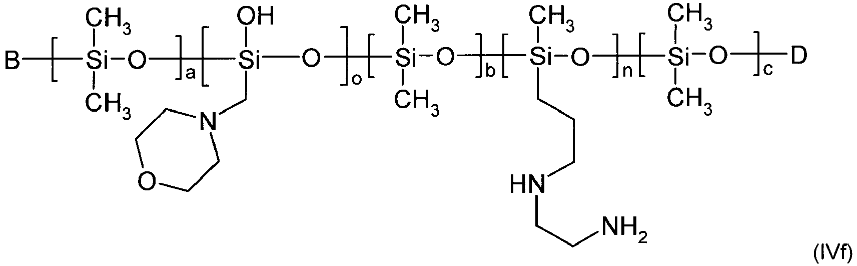 enthält, in denen
B für eine Gruppe -OH, -O-Si(CH₃)₃,-O-Si(CH₃)₂OH -O-Si(CH₃)₂OCH₃ steht,
D für eine Gruppe -H, -Si(CH₃)₃,-Si(CH₃)₂OH, -Si(CH₃)₂OCH₃ steht,
a, b und c für ganze Zahlen zwischen 0 und 1000 stehen, mit der Maßgabe a + b + c > 0
m, n und o für ganze Zahlen zwischen 1 und 1000 stehen,
mit der Maßgabe, daß mindestens eine der Bedingungen B = -OH bzw. D = -H erfüllt ist.

7. Verfahren nach einem der Ansprüche 1 bis 6, **dadurch gekennzeichnet, daß** im Schritt (i) ein Vorbehandlungsmittel aufgetragen wird, welches - bezogen auf sein Gewicht - 0,00001 bis 10 Gew.-%, vorzugsweise 0,0001 bis 7,5 Gew.-%, besonders bevorzugt 0,001 bis 5 Gew.-%, weiter bevorzugt 0,01 bis 3 Gew.-% und insbesondere 0,1 bis 1 Gew.-% 4-morpholinomethyl-substituierte(s) Silikon(e) enthält.

8. Verfahren nach einem der Ansprüche 1 bis 7, **dadurch gekennzeichnet, daß** im Schritt (i) ein Vorbehandlungsmittel aufgetragen wird, welches in Form einer Mikroemulsion vorliegt, welche Fettalkohol(e) enthält.

9. Verfahren nach einem der Ansprüche 1 bis 8, **dadurch gekennzeichnet, daß** im Schritt (i) ein Vorbehandlungsmittel aufgetragen wird, welches - bezogen auf sein Gewicht - 0,00001 bis 5 Gew.-%, vorzugsweise 0,0001 bis 3,5 Gew.-%, besonders bevorzugt 0,001 bis 2 Gew.-%, weiter bevorzugt 0,01 bis 1 Gew.-% und insbesondere 0,1 bis 0,5 Gew.-% verzweigtes, ethoxyliertes Tridecanol (INCI-Bezeichnung: Trideceth-5) oder α-iso-Tridecyl-ω-hydroxypolyglycolether (INCI-Bezeichnung: Trideceth-10) oder deren Mischungen enthält.

10. Verfahren nach einem der Ansprüche 1 bis 9, **dadurch gekennzeichnet, daß** die Fasern in Schritt (ii) getrocknet werden.

11. Verfahren nach einem der Ansprüche 1 bis 10, **dadurch gekennzeichnet, daß** die Fasern in Schritt (iii) mit einem Umformungsmittel behandelt werden, welches - bezogen auf das Gewicht des Umformungsmittels - 0,05 bis 20 Gew.-%, vorzugsweise 0,1 bis 15 Gew.-%, weiter bevorzugt 0,25 bis 10 Gew.-%, noch weiter bevorzugt 0,5 bis 5 Gew.-%, besonders bevorzugt 0,75 bis 3 Gew.-% und insbesondere 1 bis 2 Gew.-% Natriumhydroxid enthält.

12. Verfahren nach einem der Ansprüche 1 bis 11, **dadurch gekennzeichnet, daß** das Shampoo im Behandlungsschritt (v) Phenolsulphonthalein als pH Indikator enthält.

13. Verfahren nach einem der Ansprüche 1 bis 12, **dadurch gekennzeichnet, daß** die Fasern in Schritt (vi) einer Wärmebehandlung bei einer Temperatur von 50°C bis 350°C (vorzugsweise 80°C bis 280°C, besonders bevorzugt 100°C bis 250°C, weiter bevorzugt 140°C bis 220°C) unterworfen werden.

## Claims

1. A method for shaping, in particular for straightening, keratin-containing fibers, in particular human hair, in which
(i) a pretreatment agent containing amino-functional silicone(s) having terminal hydroxy group(s) is applied to the keratin fibers and left there,
(ii) the fibers are optionally dried,
(iii) the fibers are treated in the moist or dried state with a shaping agent which, based on the weight of the shaping agent, contains from 0.05 to 20 wt.% of alkali metal hydroxide(s),
(iv) the fibers are optionally straightened using a comb or a brush during the shaping treatment,
(v) the fibers are shampooed using a shampoo which has a pH of from 2.5 to 6.5, rinsed and neutralized,
(vi) the fibers are optionally subsequently additionally mechanically deformed under the action of heat.

2. The method according to claim 1, **characterized in that**, in step (i), a pretreatment agent is applied which, based on the weight thereof, contains from 0.01 to 5 wt.%, preferably from 0.025 to 2.5 wt.%, more preferably from 0.05 to 1.5 wt.%, even more preferably from 0.075 to 1 wt.% and in particular from 0.1 to 0.25 wt.%, of at least one silicone of formula (I) 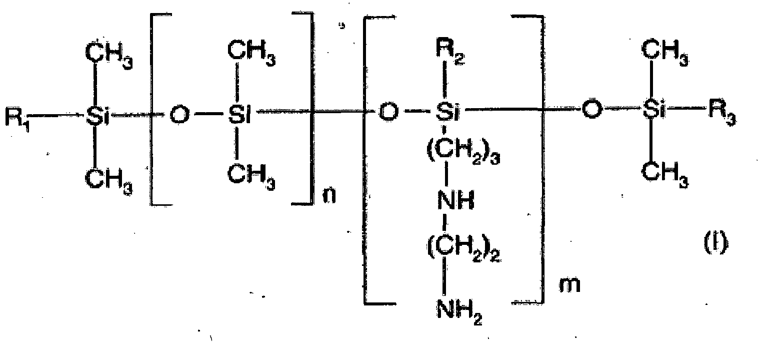 in which
- m and n denote numbers which are selected such that the sum (n + m) is in the range of from 1 to 1000,
- n is a number in the range of from 0 to 999 and m is a number in the range of from 1 to 1000,
- R1, R2 and R3, which are identical or different, denote a hydroxy group or a C₁₋₄ alkoxy group,
- at least one of the groups R1 to R3 denoting a hydroxy group.

3. The method according to either claim 1 or claim 2, **characterized in that**, in step (i), a pretreatment agent is applied which, based on the weight thereof, contains from 0.01 to 5 wt.%, preferably from 0.025 to 2.5 wt.%, more preferably from 0.05 to 1.5 wt.%, even more preferably from 0.075 to 1 wt.% and in particular from 0.1 to 0.25 wt.%, of at least one silicone of formula (II) 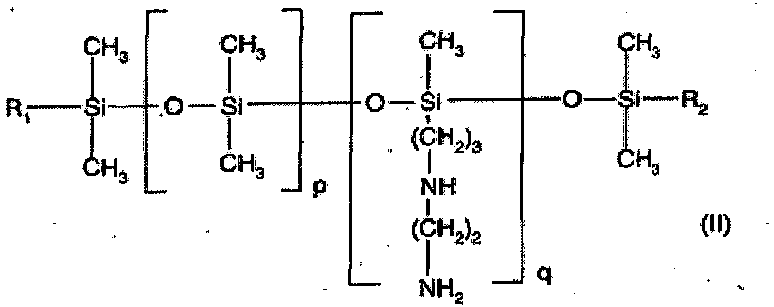 in which
- p and q denote numbers which are selected such that the sum (p + q) is in the range of from 1 to 1000,
- p is a number in the range of from 0 to 999 and q is a number in the range of from 1 to 1000,
- R1 and R2, which are different, denote a hydroxy group or a C₁₋₄ alkoxy group, at least one of the groups R1 to R2 denoting a hydroxy group.

4. The method according to one of claims 1 to 3, **characterized in that**, in step (i), a pretreatment agent is applied which, based on the weight thereof, contains from 0.01 to 5 wt.%, preferably from 0.025 to 2.5 wt.%, more preferably from 0.05 to 1.5 wt.%, even more preferably from 0.075 to 1 wt.% and in particular from 0.1 to 0.25 wt.%, of at least one silicone of formula (III): 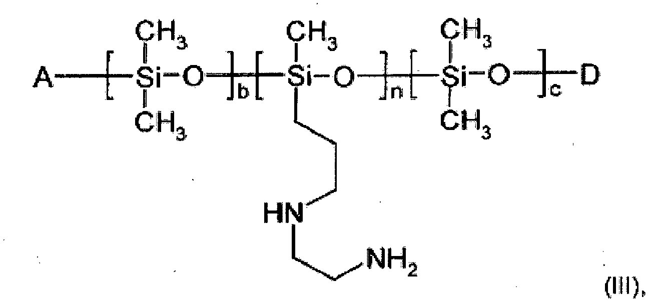 in which
A represents a -OH, -O-Si(CH₃)₃, -O-Si(CH₃)₂OH or -O-Si(CH₃)₂OCH₃ group,
D represents a -H, -Si(CH₃)₃, -Si(CH₃)₂OH or -Si(CH₃)₂OCH₃ group,
b, n and c represent integers of between 0 and 1000,
with the provisos
- n > 0 and b + c > 0
- at least one of the conditions A = -OH or D = -H is met.

5. The method according to one of claims 1 to 4, **characterized in that**, in step (i), a pretreatment agent is applied which, based on the weight thereof, contains from 0.01 to 5 wt.%, preferably from 0.025 to 2.5 wt.%, more preferably from 0.05 to 1.5 wt.%, even more preferably from 0.075 to 1 wt.% and in particular from 0.1 to 0.25 wt.%, of at least one 4-morpholinomethyl-substituted silicone of formula (IV): 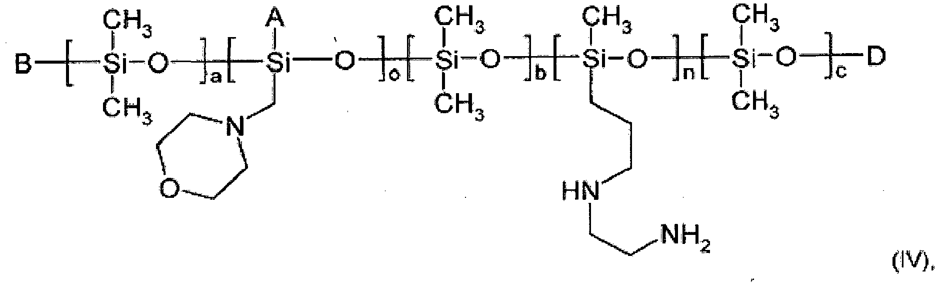 in which
A represents a structural unit (I) bound by an -O- 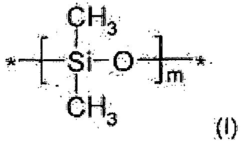 or represents an oligomeric or polymeric functional group bound by an -O- and containing structural units of formula (I), or represents -OH,
* represents a terminal group B (Si-bound) or D (O-bound),
B represents a -OH, -O-Si(CH₃)₃, -O-Si(CH₃)₂OH or -O-Si(CH₃)₂OCH₃ group,
D represents a -H, -Si(CH₃)₃, -Si(CH₃)₂OH or -Si(CH₃)₂OCH₃ group,
a, b and c represent integers of between 0 and 1000, with the proviso a + b + c > 0
m, n and o represent integers of between 1 and 1000,
with the proviso that at least one of the conditions B = -OH or D = -H is met.

6. The method according to one of claims 1 to 5, **characterized in that**, in step (i), a pretreatment agent is applied which, based on the weight thereof, contains from 0.01 to 5 wt.%, preferably from 0.025 to 2.5 wt.%, more preferably from 0.05 to 1.5 wt.%, even more preferably from 0.075 to 1 wt.% and in particular from 0.1 to 0.25 wt.%, of at least one 4-morpholinomethyl-substituted silicone of formulas (IVa) or (IVf) 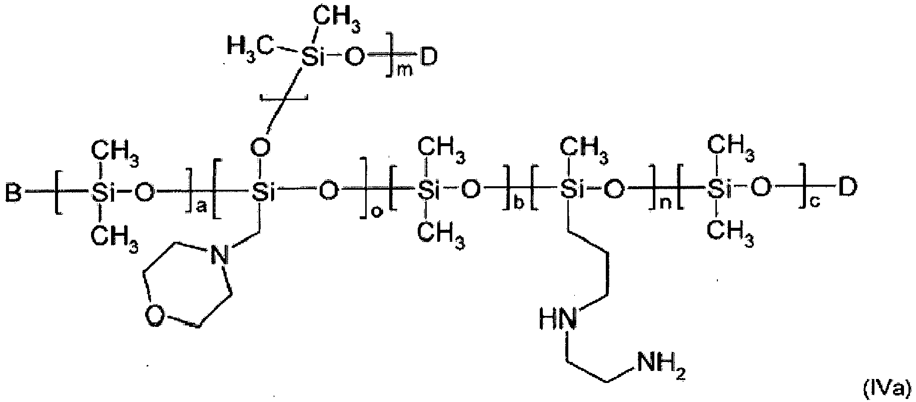 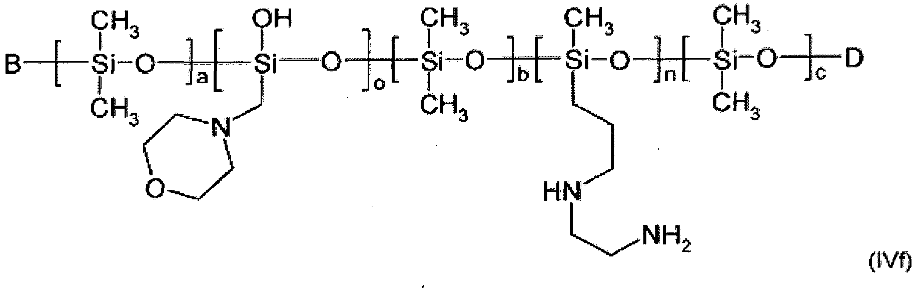 in which
B represents a -OH, -O-Si(CH₃)₃, -O-Si(CH₃)₂OH, -O-Si(CH₃)₂OCH₃ group,
D represents a -H, -Si(CH₃)₃, -Si(CH₃)₂OH, -Si(CH₃)₂OCH₃ group,
a, b and c represent integers of between 0 and 1000, with the proviso a + b + c > 0
m, n and o represent integers of between 1 and 1000,
with the proviso that at least one of the conditions B = -OH or D = -H is met.

7. The method according to one of claims 1 to 6, **characterized in that**, in step (i), a pretreatment agent is applied which, based on the weight thereof, contains from 0.00001 to 10 wt.%, preferably from 0.0001 to 7.5 wt.%, particularly preferably from 0.001 to 5 wt.%, more preferably from 0.01 to 3 wt.% and in particular from 0.1 to 1 wt.%, of 4-morpholinomethyl-substituted silicone(s).

8. The method according to one of claims 1 to 7, **characterized in that**, in step (i), a pretreatment agent is applied which is in the form of a microemulsion which contains fatty alcohol(s).

9. The method according to one of claims 1 to 8, **characterized in that**, in step (i), a pretreatment agent is applied which, based on the weight thereof, contains from 0.00001 to 5 wt.%, preferably from 0.0001 to 3.5 wt.%, particularly preferably from 0.001 to 2 wt.%, more preferably from 0.01 to 1 wt.% and in particular from 0.1 to 0.5 wt.%, of branched, ethoxylated tridecanol (INCI designation: Trideceth-5) or α-iso-tridecyl-ω-hydroxy polyglycol ether (INCI designation: Trideceth-10) or mixtures thereof.

10. The method according to one of claims 1 to 9, **characterized in that** the fibers are dried in step (ii).

11. The method according to one of claims 1 to 10, **characterized in that** the fibers are treated in step (iii) with a shaping agent which, based on the weight of the shaping agent, contains from 0.05 to 20 wt.%, preferably from 0.1 to 15 wt.%, more preferably from 0.25 to 10 wt.%, even more preferably from 0.5 to 5 wt.%, particularly preferably from 0.75 to 3 wt.% and in particular from 1 to 2 wt.%, of sodium hydroxide.

12. The method according to one of claims 1 to 11, **characterized in that** the shampoo in treatment step (v) contains phenolsulfonphthalein as a pH indicator.

13. The method according to one of claims 1 to 12, **characterized in that** the fibers are subjected in step (vi) to heat treatment at a temperature of from 50°C to 350°C (preferably from 80°C to 280°C, particularly preferably from 100°C to 250°C, more preferably from 140°C to 220°C).

## Revendications

1. Procédé de mise en forme, notamment de lissage, de fibres de kératine, en particulier de cheveux humains, dans lequel
(i) un agent de prétraitement, contenant au moins une silicone à fonction amino comportant au moins un groupe hydroxy terminal, est appliqué sur les fibres de kératine où il est laissé,
(ii) les fibres sont éventuellement séchées,
(iii) les fibres sont traitées à l'état humide ou sec avec un agent de mise en forme qui contient, sur la base du poids de l'agent de mise en forme, de 0,05 à 20% en poids d'au moins un hydroxyde de métal alcalin,
(iv) les fibres sont éventuellement lissées au moyen d'un peigne ou d'une brosse pendant l'opération de mise en forme,
(v) les fibres sont shampooinées, rincées et neutralisées avec un shampooing ayant un pH de 2,5 à 6,5,
(vi) puis les fibres sont en outre éventuellement déformées mécaniquement sous l'action de la chaleur.

2. Procédé selon la revendication 1, **caractérisé en ce que** l'on applique à l'étape (i) un agent de prétraitement qui contient, sur la base de son poids, de 0,01 à 5% en poids, de préférence de 0,025 à 2,5% en poids, plus préférablement de 0,05 à 1,5% en poids, encore plus préférablement de 0,075 à 1% en poids et notamment de 0,1 à 0,25% en poids, d'au moins une silicone de la formule (I) 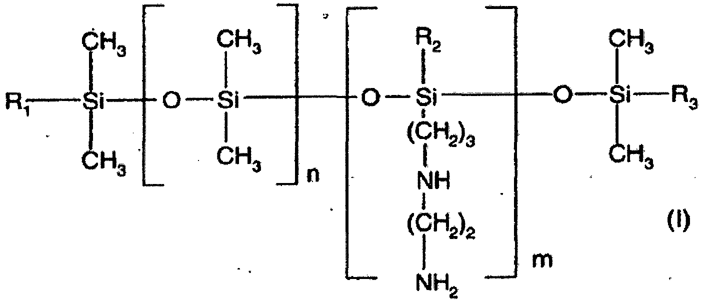 dans laquelle
- m et n sont des nombres choisis de telle sorte que la somme (n + m) soit dans la gamme de 1 à 1000,
- n est un nombre dans la gamme de 0 à 999 et m est un nombre dans la gamme de 1 à 1000,
- R1, R2 et R3, qui identiques ou différents, représentent un groupe hydroxy ou un groupe alcoxy en C₁ à C₄,
- au moins un des groupes R1 à R3 représentant un groupe hydroxy.

3. Procédé selon l'une des revendications 1 ou 2, **caractérisé en ce que** l'on applique à l'étape (i) un agent de prétraitement qui contient, sur la base de son poids, de 0,01 à 5% en poids, de préférence de 0,025 à 2,5% en poids, plus préférablement de 0,05 à 1,5% en poids, encore plus préférablement de 0,075 à 1% en poids et notamment de 0,1 à 0,25% en poids, d'au moins une silicone de la formule (II) 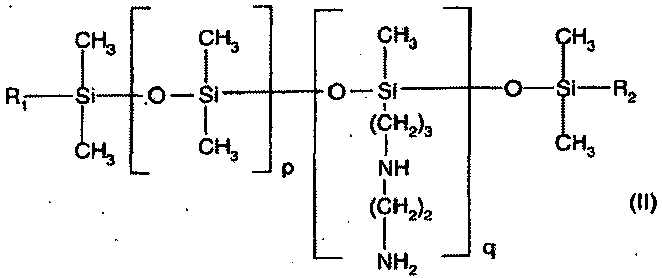 dans laquelle
- p et q sont des nombres choisis de telle sorte que la somme (p + q) soit dans la gamme de 1 à 1000,
- p est un nombre dans la gamme de 0 à 999 et q est un nombre dans la gamme de 1 à 1000,
- R1 et R2, qui sont différents, représentent un groupe hydroxy ou un groupe alcoxy en C₁ à C₄, au moins un des groupes R1 à R2 est un groupe hydroxy.

4. Procédé selon l'une des revendications 1 à 3, **caractérisé en ce que** l'on applique à l'étape (i) un agent de prétraitement qui contient, sur la base de son poids, de 0,01 à 5% en poids, de préférence de 0,025 à 2,5% en poids, plus préférablement de 0,05 à 1,5% en poids, encore plus préférablement de 0,075 à 1% en poids et notamment de 0,1 à 0,25% en poids, d'au moins une silicone de la formule (III) : 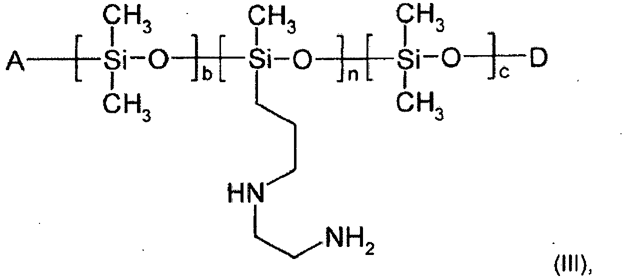 dans laquelle
A représente un groupe -OH, -O-Si(CH₃)₃, -O-Si(CH₃)₂OH, -O-Si(CH₃)₂OCH₃,
D représente un groupe -H, -Si(CH₃)₃, -Si(CH₃)₂OH, -Si(CH₃)₂OCH₃,
b, n et c sont des nombres entiers compris entre 0 et 1000,
pourvu que
- n > 0 et b + c > 0
- au moins une des conditions A = -OH ou D = -H = soit remplie.

5. Procédé selon l'une des revendications 1 à 4, **caractérisé en ce que** l'on applique à l'étape (i) un agent de prétraitement qui contient, sur la base de son poids, de 0,01 à 5% en poids, de préférence de 0,025 à 2,5% en poids, plus préférablement de 0,05 à 1,5% en poids, encore plus préférablement de 0,075 à 1% en poids et notamment de 0,1 à 0,25% en poids, d'au moins une silicone 4-morpholinométhyl-substituée de la formule (IV) : 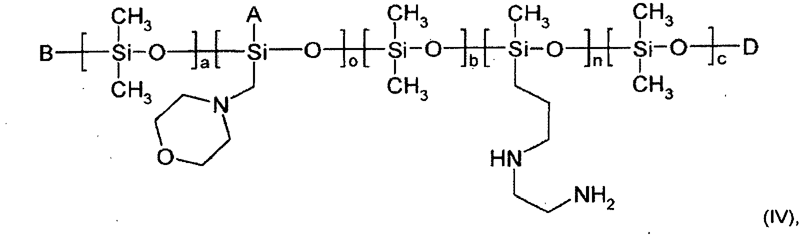 dans laquelle
A représente un motif structurel (I) lié par un -O- 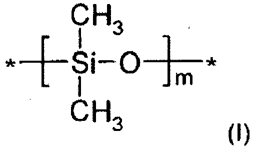 ou un radical oligomère ou polymère, lié par un -O-, contenant des motifs structuraux des formules (I) ou représente un groupe -OH,
* représente un groupe d'extrémité B (lié par Si) ou D (lié par O),
B représente un groupe -OH, -O-Si(CH₃)₃, -O-Si(CH₃)₂OH, -O-Si(CH₃)₂OCH₃,
D représente un groupe -H, -Si(CH₃)₃, -Si(CH₃)₂OH, -Si(CH₃)₂OCH₃,
a, b et c sont des nombres entiers compris entre 0 et 1000, pourvu que a + b + c > 0,
m, n et o sont des nombres entiers compris entre 1 et 1000,
pourvu qu'au moins une des conditions B = -OH ou D = -H soit satisfaite.

6. Procédé selon l'une des revendications 1 à 5, **caractérisé en ce que** l'on applique à l'étape (i) un agent de prétraitement qui contient, sur la base de son poids, de 0,01 à 5% en poids, de préférence de 0,025 à 2,5% en poids, plus préférablement de 0,05 à 1,5% en poids, encore plus préférablement de 0,075 à 1% en poids et notamment de 0,1 à 0,25% en poids, d'au moins une silicone 4-morpholinométhyl-substituée des formules (IVa) ou (IVf) 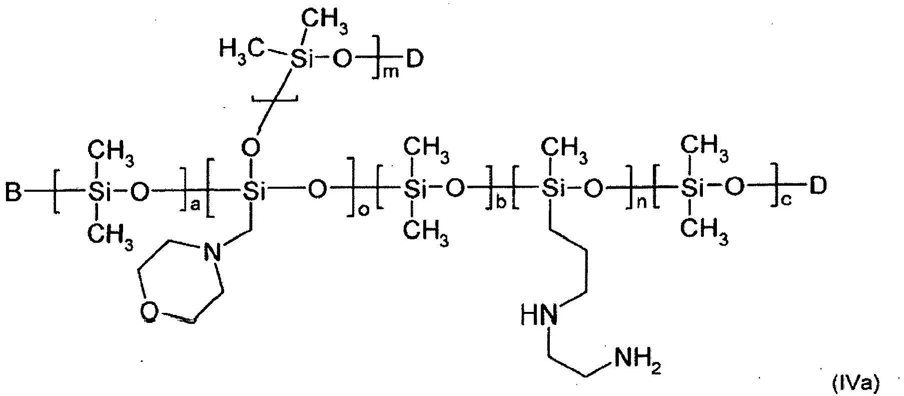 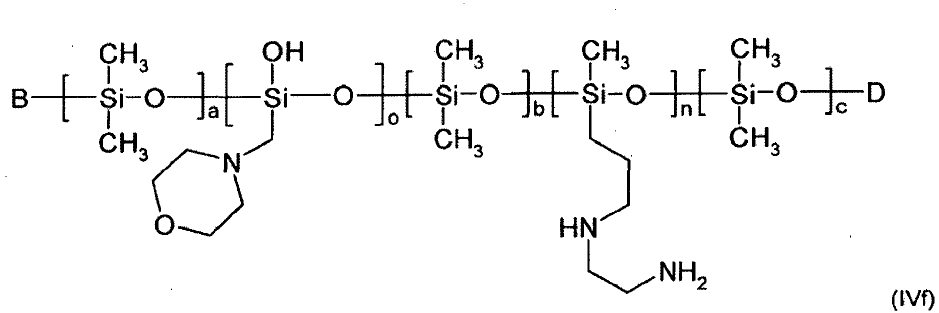 dans lesquelles
B représente un groupe -OH, -O-Si(CH₃)₃, -O-Si(CH₃)₂OH, -O-Si(CH₃)₂OCH₃,
D est un groupe -H, -Si(CH₃)₃, -Si(CH₃)₂OH, -Si(CH₃)₂OCH₃,
a, b et c sont des nombres entiers compris entre 0 et 1000, pourvu que a + b + c > 0
m, n et o sont des nombres entiers compris entre 1 et 1000,
pourvu qu'au moins une des conditions B = -OH et D = -H soit satisfaite.

7. Procédé selon l'une des revendications 1 à 6, **caractérisé en ce que** l'on applique à l'étape (i) un agent de prétraitement qui contient, sur la base de son poids, de 0,00001 à 10% en poids, de préférence de 0,0001 à 7,5% en poids, de façon particulièrement préférée de 0,001 à 5% en poids, plus préférablement de 0,01 à 3% en poids et notamment de 0,1 à 1% en poids, d'au moins une silicone 4-morpholinométhyl-substituée.

8. Procédé selon l'une des revendications 1 à 7, **caractérisé en ce que** l'on applique à l'étape (i) un agent de prétraitement qui se présente sous la forme d'une microémulsion qui contient un ou plusieurs alcools gras.

9. Procédé selon l'une des revendications 1 à 8, **caractérisé en ce que** l'on applique à l'étape (i) un agent de prétraitement qui contient, sur la base de son poids, de 0,00001 à 5% en poids, de préférence de 0,0001 à 3,5% en poids, de manière particulièrement préférée de 0,001 à 2% en poids, plus préférablement de 0,01 à 1% en poids et notamment de 0,1 à 0,5% en poids, de tridécanol éthoxylé ramifié (nom INCI : Trideceth-5) ou de α-iso-tridécyle-ω-hydroxypolyglycoléther (nom INCI : Trideceth-10) ou des mélanges de ceux-ci.

10. Procédé selon l'une des revendications 1 à 9, **caractérisé en ce que** les fibres sont séchées à l'étape (ii).

11. Procédé selon l'une des revendications 1 à 10, **caractérisé en ce que** les fibres sont traitées à l'étape (iii) avec un agent de mise en forme qui contient, sur la base du poids de l'agent de mise en forme, de 0,05 à 20% en poids, de préférence de 0,1 à 15% en poids, plus préférablement de 0,25 à 10% en poids, encore plus préférablement de 0,5 à 5% en poids, de manière particulièrement préférée de 0,75 à 3% en poids et notamment de 1 à 2% en poids, d'hydroxyde de sodium.

12. Procédé selon l'une des revendications 1 à 11, **caractérisé en ce que** le shampooing de l'étape de traitement (v) contient comme indicateur de pH de la phénolsulfonphthaléine.

13. Procédé selon l'une des revendications 1 à 12, **caractérisé en ce que** les fibres sont soumises à l'étape (vi) à un traitement thermique à une température de 50°C à 350°C (de préférence 80°C à 280°C, de manière particulièrement préférée de 100°C à 250°C, plus préférablement de 140°C à 220°C).
